(19) Europäisches Patentamt
European Patent Office
Office européen des brevets

(11) Publication number: **0 124 934**
**B1**

(12) **EUROPEAN PATENT SPECIFICATION**

(45) Date of publication of patent specification: **20.11.86**

(51) Int. Cl.$^4$: **C 07 J 41/00**

(21) Application number: **84200595.1**

(22) Date of filing: **27.04.84**

(54) **17-(Isocyano-sulfonylmethylene)-steroids, 17-(formamido-sulfonylmethylene)-steroids and their preparation.**

(30) Priority: **29.04.83 EP 83200616**

(43) Date of publication of application:
**14.11.84 Bulletin 84/46**

(45) Publication of the grant of the patent:
**20.11.86 Bulletin 86/47**

(84) Designated Contracting States:
**AT BE CH DE FR GB IT LI LU NL SE**

(56) References cited:
**EP-A-0 007 672**
**EP-A-0 023 856**
**GB-A-2 079 756**

(73) Proprietor: **Gist - Brocades N.V.**
**Wateringseweg 1 P.O. Box 1**
**NL-2600 MA Delft (NL)**

(72) Inventor: **Van Leusen, Albert Mattheus**
**De Savornin Lohmanlaan 23**
**NL-9722 HC Groningen (NL)**
Inventor: **Van Leusen, Adriaan Marinus**
**Binnensingel 8**
**NL-9951 GD Winsum (NL)**

(74) Representative: **Van der Straaten, Jan Anthony et al**
**c/o GIST-BROCADES N.V. Patents and Trademarks Department Martinus Nijhofflaan 2**
**P.O. Box 1**
**NL-2600 MA Delft (NL)**

**EP 0 124 934 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to 17-(isocyano-sulfonylmethylene)-steroids and a process for the preparation of these compounds. Furthermore the invention relates to 17-(formamido-sulfonylmethylene)-steroids which are intermediates in the preparation of the 17-(isocyano-sulfonylmethylene)-steroids, and a process for the preparation of these compounds.

Steroids are used on a large scale as constituents of many kinds of pharmaceutical compositions. Dependant on the substituent pattern of the carbon-skeleton the steroids can be divided into a number of main classes. An important main class of steroids is formed by the cortico-steroids. The natural representatives of the corticosteroids are usually produced by the adrenal gland. Corticosteroids are characterized by the presence of a 3-keto group, a delta$^4$ bond, an 11-beta-hydroxy group, a 17-alpha-hydroxy group and a 17-beta-hydroxy-acetyl side chain.

During a long time corticosteroids were made by chemical degradation of gall acids as cholic acid, desoxycholic acid and glycocholic acid. Afterwards, hecogenin, which could be isolated from plants, particularly from numerous Agave species, became an important raw material too. Since the possibility of introduction of an 11-hydroxy group by microbiological methods, diosgenin, which could be isolated from numerous Dioscoreacaea species, and stigmasterol, usually isolated from the phytosterol mixture from soya or calabar beans, have become the most important raw material for the preparation of corticosteroids.

Much attention has been given to new, cheaper raw materials for the synthesis of pharmaceutically active steroids. Therefore, the degradation of the abundant soya bean derived sterols sitosterol and campesterol by microbiological methods into 17-oxo-steroids was extensively investigated. As a result thereof 17-oxo-steroids are readily available now at low prices, which makes these compounds, together with the possibility of the introduction of an 11-hydroxy group by microbiological methods, ideal starting materials for corticosteroids synthesis.

A number of chemical synthesis for the construction of the corticosteroids side chain from 17-oxo-steroids is known. For instance in J. Org. Chem., *44*, 1582 (1979) a method is described which uses a sulfenate-sulfoxide rearrangement for the introduction of the 17-(dihydroxyacetone) side chain. Another route is described in J.C.S. Chem. Comm, *1981*, 775, in which the reaction of 17-oxo-steroids with ethyl isocyanoacetate is described, followed by a number of other reactions, which ultimately result in the dihydroxyacetone side chain of corticosteroids. Other syntheses of the corticosteroid side chain or of compounds which can be used as precursors therefore are described in J.C.S. Chem. Comm., *1981*, 774, J.C.S. Chem. Comm. *1982*, 551, Chem. Ber., *113*, 1184 (1980), and J. Org. Chem., 1982, 2993.

The present invention now relates to the preparation of intermediates for the preparation of 21-hydroxy-20-keto-delta$^{16}$-steroids and 20-keto-delta$^{16}$-steroids, which compounds on their turn can be used for the preparation of pharmaceutically active compounds, especially corticosteroids.

More particularly, the invention concerns 17-(isocyanosulfonylmethylene)-steroids of the general formula

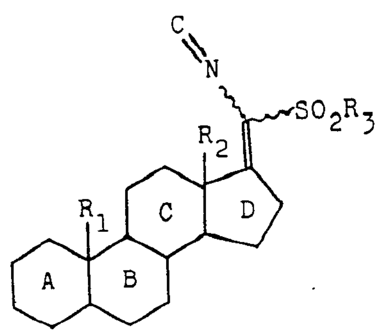

I

in which $R_1$ represents a hydrogen atom or an alkyl group having 1 to 4 carbon atoms, preferably a methyl group, or is absent in the case of a double bond between $C_{10}$ and $C_1$, $C_5$ or $C_9$, $R_2$ represents a hydrogen atom or an alkyl group having 1 to 4 carbon atoms, preferably a methyl group, $R_3$ represents an alkyl or dialkylamino group or an aryl group substituted or not substituted by one or more halogen atoms, alkyl or alkoxy groups, and the rings A, B, C and D optionally contain one or more double bonds, are substituted or not substituted by one or more hydroxy groups, amino groups, oxygen atoms, halogen atoms or alkyl, alkylene, alkoxy or alkoxyalkoxy groups and are disubstituted or not disubstituted by one or more epoxy groups, methylene groups, alkylenedioxy, alkylenedithio or alkylene-oxythio groups.

When $R_3$ represents an alkyl group, suitable alkyl groups are straight or branched alkyl groups having 1 to 10 carbon atoms.

When $R_3$ represents a dialkylamino group, suitable dialkylamino groups are dialkylamino groups wherein the alkyl groups are the same or different and contain 1—8 carbon atoms, preferably 1—4 carbon atoms, or a dialkylamino group wherein the alkyl groups together with the nitrogen atom form a heterocyclic ring which optionally may contain an oxygen atom, the ring containing up to 8 ring atoms. Preferred dialkylamino groups are dimethylamino, diethylamino, pyrolidine and morfoline.

When $R_3$ represents an aryl group, suitable groups are phenyl and naphthyl groups substituted or not substituted by a halogen atom, one or more alkyl groups or an alkoxy group, preferably a phenyl, p-methoxyphenyl or p-methylphenyl group.

When the rings A, B, C and D contain one or more double bonds, these double bonds are preferably present between $C_1$ and $C_2$, $C_3$ and $C_4$, $C_4$ and $C_5$, $C_5$ and $C_6$, $C_6$ and $C_7$, $C_9$ and $C_{10}$, $C_9$ and $C_{11}$ and/or $C_{11}$ and $C_{12}$. More preferably the double bond is present between $C_4$ and $C_5$ and/or $C_9$ and $C_{11}$.

When two or more double bonds are present especially the following systems are preferred: $C_3$—$C_4$ and $C_5$—$C_6$, $C_4$—$C_5$ and $C_6$—$C_7$, $C_1$—$C_2$ and $C_4$—$C_5$, $C_1$—$C_2$, $C_3$—$C_4$ and $C_5$—$C_{10}$ and $C_1$—$C_2$, $C_4$—$C_5$ and $C_6$—$C_7$. Preferably there is also a double bond between $C_9$ and $C_{11}$.

When the rings A, B, C and D are substituted by a hydroxy group, suitable groups are 3-, 9-, 11-, 12- or 14-hydroxy groups, preferably a 3- or 9-hydroxy group.

When the rings A, B, C and D are substituted by an amino group, suitable amino groups are 3-alkylamino groups, preferably containing 1—4 carbon atoms, 3-dialkylamino groups wherein the alkyl groups are the same or different, each alkyl group preferably containing 1—4 carbon atoms, or a dialkyl amino group wherein the alkyl groups together with the nitrogen atom form a heterocyclic ring, preferably containing 1—8 ring atoms, which ring optionally may contain an oxygen atom. Particularly preferred are dimethylamino, diethylamino, pyrolidine and morfoline.

When the rings A, B, C and D are substituted by an oxygen atom this oxygen atom is preferably present at $C_3$, $C_{11}$ or $C_{12}$.

When the rings A, B, C and D are substituted by a halogen atom, suitable halogen atoms are 6-, 9- or 11-fluorine, chlorine or bromine atoms, preferably 6- or 9-fluorine or chlorine atoms.

When the rings A, B, C and D are substituted by an alkyl group, suitable alkyl groups are 1-, 2-, 6-, 7- or 16-methyl groups, preferably 1 or 6-methyl.

When the rings A, B, C and D are substituted by an alkoxy group, suitable alkoxy groups are 3-, 9-, 11- or 12-alkoxy groups containing 1—4 carbon atoms, preferably 3-, 9-, or 11-methoxy or ethoxy groups.

When the rings A, B, C and D are substituted by an alkoxyalkoxy group, suitable groups are 3- or 11-methoxymethoxy, methoxyethoxy or tetrahydropyranyloxy.

When the rings A, B, C and D are disubstituted, suitable substituents are epoxy groups at $C_1$ and $C_2$ or $C_9$ and $C_{11}$ or a methylene group attached to $C_1$ and $C_2$ or a 3,3-alkylenedioxy, a 3,3-alkylenedithio or a 3,3-alkyleneoxythio group. The alkylene group preferably contains 2 or 3 carbon atoms.

More particularly the invention relates to compounds in which $R_1$ and $R_2$ represent methyl or in which $R_1$ is absent, which are substituted by halogen, especially fluorine, or hydroxy at $C_9$ and a hydroxy or keto group at $C_{11}$, or containing functional groups, as a double bond or epoxy group between $C_9$ and $C_{11}$, which can be converted by methods known in the art into the groups mentioned before, and which contain a keto group at $C_3$ and double bonds between $C_1$ and $C_2$ and/or $C_4$ and $C_5$, or containing functional groups which can be converted into the keto group and double bonds mentioned before.

The invention aims at the preparation of 17-(isocyanosulfonylmethylene-steroids, because these compounds are valuable intermediate in the preparation of 21-hydroxy-20-keto-delta$^{16}$-steroids and 20-keto-delta$^{16}$-steroids as described in the simultaneously filed applications EP—A—0127216 and 0127217 entitled: "New process for the preparation of 21-hydroxy-20-keto-delta$^{16}$-steroids and new intermediate compounds formed in this process" and "New process for the preparation of 20-keto-delta$^{16}$-steroids and new intermediate compounds formed in this process". These compounds can be converted into pharmaceutically active steroids by methods known in the art.

The before-mentioned 21-hydroxy-20-keto-delta$^{16}$-steroids are prepared by reaction of the 17-(isocyano-sulfonylmethylene)-steroids with an aldehyde and an alcohol under basic conditions, followed by hydrolysis of the intermediate 17-(2-alkoxy-3-oxazolin-4-yl)-delta$^{16}$-steroids. The before-mentioned 20-keto-delta$^{16}$-steroids are prepared by reaction of the 17-(isocyano-sulfonylmethylene)-steroids with an alkylating agent under basic conditions, followed by hydrolysis of the intermediate 20-isocyano-20-sulfonyl-delta$^{16}$-steroids.

The invention also relates to a process for the preparation of 17-(isocyano-sulfonylmethylene)-steroids by reacting a 17-oxo-steroid with a sulfonylmethylisocyanide and then dehydrating the resulting formamide to the corresponding isocyanide. Such a process is known. For instance EP—A—0 007 672 discloses the said process applied to numerous ketones. It has now been found that 17-(formamido-sulfonylmethylene)-steroids and 17-(isocyano-sulfonylmethylene)-steroids could be prepared according to the process as described in EP—A—0 007 672, starting with 17-oxo-steroids.

Therefore the invention also relates to a process for the preparation of 17-(isocyano-sulfonylmethylene)-steroids by reacting a ketone with a sulfonylmethylisocyanide, followed by dehydration of the resulting formamide, characterized in that the ketone is a 17-oxo-steroid.

In this connection the following is observed. The above mentioned European patent application contains one example (example 60) in which a steroid is used for the preparation of an alpha,beta-unsaturated sulphonylmethyl formamide, and also the dehydration of this formamide to the corresponding isocyanide is described (example 26). In these examples the starting material was a 3-oxo-steroid.

However, as the 3-oxo-group of a steroid is more reactive than the 17-oxo-group, mainly due to steric reasons, it is not predictable for a man skilled in the art that these reactions also could be performed at the 17-oxo-group, especially not because of the known difficulties of reactions of p-methylphenylsulfonylmethyl-isocyanide in other type of reactions with sterically hindered ketones.

It is remarked in this respect that reactions of p-methylphenylsulfonylmethylisocyanides with 17-oxo-steroids were known already, as appears from for instance Tetrahedron *31*, 2151 and 2157. In these publications the preparation of 17-alpha and 17-beta cyano steroids is described. As a result of the already before mentioned steric hindrance of the 17-oxo-group, the reaction with p-methylphenylsulfonylmethylisocyanide to the 17-cyano steroids could only be performed by using drastic reaction conditions.

It is generally believed that the above-mentioned alpha,beta-unsaturated formamides, or more accurately their deprotonated anions, are intermediates in the formation of the cyano compounds.

Using the drastic reaction conditions, necessary for the first step in the reaction scheme in view of the steric hindrance of the 17-oxo-group, one would expect that the formamides, once formed, would react immediately further into the before mentioned cyano-compounds, and thus, isolation of the alpha, beta-unsaturated formamides would be impossible.

It was therefore surprising that it was still possible to isolate the desired alpha, beta-unsaturated formamides instead of the cyanides which would be expected. This could be reached mainly by using sufficiently low temperatures, i.e. temperatures below −20°C, preferably at −40°C.

The invention also relates to the intermediate 17-(formamido-sulfonylmethylene)-steroids of the general formula:

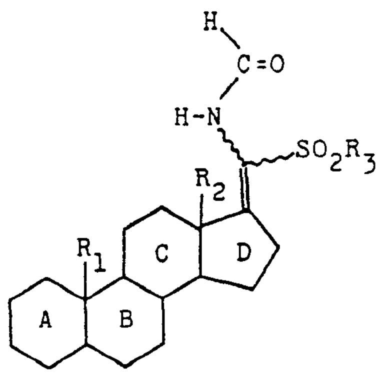

in which the substituents are as defined above.

Furthermore, the invention also relates to a process for the preparation of 17-(formamido-sulfonylmethylene)-steroids by reacting a ketone with a sulfonylmethyl isocyanide characterized in that the ketone is a 17-oxo-steroid.

As the 17-(formamido-sulfonylmethylene)-steroids are intermediates in the preparation of the 17-(isocyanosulfonylmethylene)-steroids, the invention also relates to a process for the preparation of 17-(isocyanosulfonylmethylene)-steroids, characterized in that a 17-(formamido-sulfonylmethylene)-steroid is dehydrated.

It is a further feature of the invention that both steps of the preparation process can be combined to a "one-pot-process".

If necessary, in order to obtain the desired steroids or to improve the yield, protective groups may be introduced. The protective group may be removed after the first or the second reaction step, the former is recommendable when the protective group affects unfavourably the second reaction step.

It is observed that the presence of a protective group can also be important when the isocyanides according to this invention are applied as intermediates for the preparation of 21-hydroxy-20-keto-delta$^{16}$-steroids or 20-keto-delta$^{16}$-steroids as described in the simultaneously filed applications. Therefore, it is not always necessary to remove the protective group, sometimes it is even undesired. For example, methoxy together with a double bond between $C_3$ and $C_4$ or tetrahydropyranyloxy at the 3-position are protecting groups for the 3-oxo- or 3-hydroxy group and are preferably retained until their hydrolyses during the last reaction step in the preparation of the said hydroxy-keto-steroids.

Suitable 17-oxo-steroids for the process of the invention are those 17-oxo-steroids with the general formula:

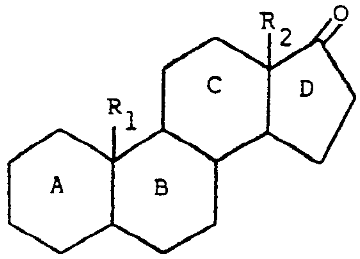

in which the steroid is as defined before. Those steorids which contain one or more groups which would interfere during the reaction have to be protected at the relevant positions. This can be done by methods known in the art. For the reaction of the 17-oxo-steroids with the sulfonylmethylisocyanides the general reaction conditions can be used as described by Schöllkopf et al. Angew. Chemie, Int. Ed., 12, 407 (1973) and by Van Leusen et al, Recl. Trav. Chim. Pays Bas *98*, 258 (1982). The temperature during the reaction has to be kept below −20°C.

Usually the reaction is carried out with a strong alkaline agent in an organic solvent, preferably in an inert gas atmosphere. Examples of useful strong alkaline agents are alkali metal alcoholates such as alkali metal t-butylates and alkali metal ethanolates, alkali metal hydrides, alkali metal amides, alkali metal alkyls and alkali metal aryls, in which the alkali metal is generally lithium, sodium or potassium and amines, preferably alkylamines. Potassium t-butoxide is preferably used.

The reaction has to be carried out at a low temperature, between −20 and −80°C, preferably between −30 and −60°C, dependant on the solvent used.

The reaction is further preferably carried out in a polar organic solvent such as tetrahydrofuran, dimethylformamide, 1,2-dimethoxyethane, hexamethylfosfortriamide, dioxane, toluene or mixtures thereof. Tetrahydrofuran is preferred. The inert gas atmosphere is preferably a nitrogen or an argon atmosphere.

It will be appreciated that in principle the group $R_3$ of the sulfonylmethylisocyanides $R_3—SO_2—CH_2—N{\equiv}C$ to be applied can be any group which does not interfere in the reaction. At least it will be possible to use those classes of sulfonylisocyanides which have been used already for this type of reactions. Examples of these classes are those compounds in which $R_3$ is aryl, alkyl or dialkylamino, whereby optionally one or more substituents can be present as described above.

Suitable sulfonylmethylisocyanides are arylsulfonylmethylisocyanides in which the aryl group is a phenyl or naphtyl group, optionally substituted by one or more halogen atoms, alkyl, alkoxy groups. Preferred arylsulfonylmethylisocyanides are phenylsulfonylmethylisocyanides in which the phenyl group is substituted or not substituted by a halogen atom, one or more alkyl groups or an alkoxy group. Particularly preferred are phenylsulfonyl-methylisocyanide and p-methylphenylsulfonylmethylisocyanide.

Any method for the preparation of the alpha, beta-unsaturated isocyanides from the corresponding formamides may be used, for example the reaction with fosforoxychloride in the presence of an amine. This reaction is preferably carried out at lower temperatures, e.g. between −50° and 25°C, preferably between −30° and −5°C. Other dehydrating agents, may however, also be used. Examples thereof are fosgene, thionyl chloride, cyanuryl chloride, alkyl and arylsulfonyl chlorides, a mixture of trifenylfosfine, carbon tetrachloride and triethyl amine, 2-chloro-3-ethylbenzoxazolium tetrafluoroborate or fosfor tri- or penta-chloride (see Ugi, Isonitril Chemistry, Acad. Press New York, *1971,* pages 10 to 16) and difosgene (see Angew. Chemie, *89*, 2671 (1977)). The dehydration is preferably carried out in the presence of an acid-binding agent, such as an amine. Examples of suitable amines are triethyl amine, substituted or unsubstituted pyridines, N-methylmorpholine, while other alkaline agents may be used too, such as potassium carbonate, sodium carbonate, potassium t-butoxide. The dehydration is preferably carried out in an inert organic solvent, such as di-, tri- or tetra-chloromethane, ethyl acetate, dioxan, tetrahydrofuran, benzene, toluene, xylene, o-dichlorobenzene, acetone, 1,2-dimethoxyethane, bis(2-methoxyethyl)-ether, dimethylformamide or 1,2-dichloroethane or mixtures thereof.

The invention is illustrated by the following examples. In these examples TosMIC represents tosylmethyl isocyanide (p-methylphenylsulfonylmethyl isocyanide).

The specific rotation of the compounds was measured using light of the sodium D line.

Example 1a

Preparation of 3-methoxy-17-(isocyano-p-methylphenylsulfonylmethylene) androsta-3,5-diene

Potassium-t-butoxide (840 mg, 7.5 mmol) was added to dry tetrahydrofuran (50 ml) whereafter the suspension was cooled to −40°C TosMIC (1.17 g, 6 mmol) was added to the suspension at −40°C. After 10

minutes stirring at this temperature 3-methoxyandrosta-3,5-dien-17-one (1.5 g, 5 mmol) was added. The mixture was stirred for two hours at −40/−30°C, followed by the addition of fosforous acid (615 mg, 7.5 mmol) at −35°C. After stirring for 10 minutes, triethylamine (7.5 ml, 54 mmol) and fosforoxytrichloride (1 ml, 11 mmol) were added at −35°C. The reaction mixture was stirred for one hour at 0°C, and poured into a mixture of 250 ml of ice water and 50 ml of brine. Extraction with $CH_2Cl_2$, drying over $MgSO_4$, evaporation in vacuum and crystallization from methanol afforded the alpha-bêta-unsaturated isocyanide (1.72 g, 3.6 mmol, 72%), m.p. 205°C (dec.); $(alpha)^{20}$ −85° (c 0.675, $CHCl_3$); IR (Nujol) 2140 (N=C), 1655, 1632, 1612 (C=C), 1600 (Ar), 1340 and 1162 ($SO_2$) $cm^{-1}$; $^1H$ NMR ($CDCl_3$) delta 0.8—3.2 (m), 0.96 (s, 3H), 2.42 (s), 3.5 (S, 3H), 5.0—5.3 (m, 2H), 7.15, 7.33, 7.64, 7.80 (ABq, 4H). Anal. calcd. for $C_{29}H_{35}NO_3S$ (477.67): C 72.92, H 7.39, N 2.93, S 6.71; found C 72.7, H 7.4, N 2.9, S 6.7.

Hydrolyses of the title compound (2.36 g, 5 mmol) into 17-(isocyano-p-methylphenylsulfonylmethylene)androst-4-ene-3-one was performed in a mixture of acetic acid (20 ml), methylene chloride (35 ml) and water (1 ml) at 25°C. After stirring for two hours the reaction mixture was poured into water, and extracted with methylene chloride. After washing with a sodium bicarbonate solution the organic layer was dried ($MgSO_4$) and evaporated in vacuum. The residue (2 g) was crystallized from methanol, m.p. 170—175°C (dec.). IR (nujol) 2140, 2130 (N=C), 1675 (C=O), 1620 (C=C), 1330, 1160 ($SO_2$) $cm^{-1}$. $^1H$ NMR ($CDCl_3$) delta 0.6—3.7 (m), 0.98 (s), 1.18 (s), 2.43 (s), 5.65 (s), 7.20, 7.35, 7.65, 7.80 (ABq).

Example 1b

Preparation of 3-methoxy-17-(formamido-p-methylphenylsulfonylmethylen)androsta-3,5-diene.

Potassium-t-butoxide (1.26 g) was added to dry tetrahydrofuran (50 ml) whereafter the suspension was cooled to −50°C. TosMIC (1.17 g) was added to the suspension. After 10 minutes stirring at this temperature 3-methoxyandrosta-3,5-dien-17-one (1.5 g) was added. The mixture was stirred for 2.5 hours at −40/−55°C, followed by the addition of 0.92 g $H_3PO_3$. The reaction mixture was stirred for 20 minutes, and poured into a mixture of 250 ml of ice water and 50 ml of brine. Extraction with $CH_2Cl_2$ drying over $MgSO_4$, evaporation in vacuum and crystallization from hexane/$CH_2Cl_2$ afforded the alpha,bêta-unsaturated formamide (1.47 g, 59%). IR ($CHCl_3$) 3396, 3367 (NH), 1699 (C=O), 1654, 1626, 1559 (C=C), 1316, 1141, ($SO_2$) $cm^{-1}$. NMR ($CDCl_3$) 0.945 (s, 6H), 2.41 (s, 3H), 3.53 (s, 3H), 5.16 (m, 2H), 7.2—8.2 (m, 6H).

Example 2

Preparation of 3-methoxy-17-(isocyano-p-methylphenylsulfonylmethylene)estra-1,3,5(10)-triene

The alpha-beta-unsaturated isocyanide was prepared according to Example 1a starting from 3-methoxyestra-1,3,5(10)-triene-17-one (1.42 g, 5 mmol). The alpha-beta-unsaturated isócyanide was precipitated from methanol as a gel. The methanol was removed and the gel was dried in vaccum. 1.62 g of the isocyanide was obtained (70%), m.p. 82—86°C (dec.). $(alpha)^{20}$ +46° (c 1.00, $CHCl_3$). IR (Nujol) 2150 (N=C), 1618, 1620 (Arom + C=C), 1390, 1342, 1162 ($SO_2$) $cm^{-1}$, $^1H$ NMR ($CDCl_3$) delta 1.1—3.2 (m), 2.42 (s, 3H), 3.70 (s, 3H), 6.53 (s, 1H), 6.68 (s, 1H), 7.00 (s, 1H), 7.21, 7.34, 7.69, 7.83 (ABq, 4H). Aanl. calcd. for $C_{28}H_{31}NO_3S$ (461.62): C 72.85, H 6.77, N 3.03, S 6.95; found: C 73.1, H 7.2, N 2.85%.

Example 3

Preparation of 17-(isocyano-p-methylphenylsulfonylmethylene)androsta-1,4-diene-3-one.

The alpha-beta-unsaturated isocyanide was prepared according to Example 1a, starting from androsta-1,4-diene-3,17-dione (1.42 g, 5 mmol). After crystallization from methanol at −20°C the isocyanide was obtained as a white solid, (1.35 g, 59%) m.p. 181—183°C (dec.). $(alpha)^{20}$ +181° (c 1.00, $CHCl_3$). IR (Nujol), 2140 (N=C), 1665 (C=O), 1630, 1610 (C=C), 1600 (Ar), 1380, 1335, 1160 ($SO_2$) $cm^{-1}$. $^1H$ NMR ($CDCl_3$): delta 0.8—3.2 (m), 1.0 (s), 1.21 (s), 2.43 (s), 6.00, 6.04, 6.20, 6.23 (2 × d 2H), 6.83, 7.00, (d, 2H,), 7.18, 7.33, 7.65, 7.78, (ABq, 4H). Anal. calcd. for $C_{28}H_{31}NO_3S$ (461.62): C 72.85, H 6.77, N 3.03, S 6.95; found C 72.6, H 6.8, N 3.0, S 7.0%.

Example 4

Preparation of 3-methoxy-17-(isocyano-p-methylphenylsulfonylmethylene)-androsta-3,5,9(11)triene.

The alpha-beta-unsaturated isocyanide was prepared according to Example 1a, starting from 3-methoxyandrosta-3,5,9 (11)-trien-17-one (1.49 g, 5 mmol). The raw product was crystallized from 40 ml of methanol. Yield: 1.84 g (77%), m.p. 162—167°C. Two further crystallizations from methylenechloride/methanol (1:4) afforded a product with a melting point 172°C (dec.). $(alpha)^{20}$ −109° (c 1.00 $CHCl_3$). IR (Nujol) 2150 (N=C), 1660, 1640, 1615, (C=C), 1605 (Ar), 1380, 1345, 1270 ($SO_2$) $cm^{-1}$. $^1H$ NMR ($CDCl_3$): delta 0.8—3.3 (m), 0.90 (s), 1.09 (s), 2.41 (s), 3.50 (s, 3H), 5.0—5.55 (m, 3H), 7.20, 7.35, 7.65, 7.80, (ABq, 4H). Anal. calcd. for $C_{29}H_{33}NO_3S$ (475.65): C 73.23, H 6.99, N 2.94, S 6.74; found: C 72.7, H 7.0, N 3.0, S 6.7%.

Example 5

Preparation of 3-methoxy-11-bêta-hydroxy-17-(isocyano-p-methylphenylsulfonylmethylene)androsta-3,5-diene.

Potassium-t-butoxide (420 mg, about 3.75 mmol) was added to dry tetrahydrofuran under nitrogen. The suspension was cooled to −40°C and then TosMIC (585 mg, 3 mmol) and 3-methoxy-11-bêta-hydroxy-androsta-3,5-dien-17-one were added. After two hours stirring at −40/−35°C fosforous acid (308 mg, 3.75

mmol) was added, followed after 10 minutes by triethylamine (7.5 ml, 54 mmol) and $POCl_3$ (1 ml, 11 mmol). The $POCl_3$ was added in such a way (during a period of about five minutes) that the temperature remained below −30°C. After two hours stirring at −30/−35°C the reaction mixture was poured into a mixture of 150 ml of water and 50 ml of brine, followed by extraction with successively 60, 30 and 30 ml of $CH_2Cl_2$. After drying, filtration through $Al_2O_3$ (act. II—III) and evaporation of the solvent an oil was obtained. Addition of 20 ml methanol yielded crystals after cooling at −20°C. Drying above NaOH at 0.2 mm Hg yielded 940 mg (76%) of the isocyano-compound, m.p. 180°C (dec.). After two further crystallisations from 10 ml of $CH_2Cl_2$/$CH_3OH$ (1:5) the resulting substance had a m.p. of 188°C (dec.). (alpha)$^{20}$ −81° (c 1.00, $CHCl_3$). IR (Nujol) 3650 (OH), 2150 (N=C), 1655, 1630, 1615, 1598 (C=C + Ar), 1340, 1165 cm$^{-1}$ ($SO_2$). $^1$H NMR ($CDCl_3$) delta 0.8—3.8 (m), 1.18 (s), 2.42 (s), 3.5 (s), 4.25—4.55 (m, 1H), 5.03 (s, 2H), 7.19, 7.33, 7.66, 7.79 (ABq, 4H). Anal. calcd. for $C_{29}H_{35}NO_4S$ (493.667): C 70.56, H 7.15, N 2.84, S 6.49: found C 70.1, H 7.2, N 2.7, S 6.5%.

### Example 6

Preparation of 3-methoxy-9-alpha-fluoro-11-beta-hydroxy-17-(isocyano-p-methylphenylsulfonylmethylene)androsta-3,5-diene.

3-Methoxy-9-alpha-fluoro-11-bêta-hydroxy-androsta-3,5-dien-17-one (835 mg, 2.5 mmol) was treated in the same way as described in Example 5. The crude isocyano compound was crystallized from 15 ml of methanol and washed with two portions of 5 ml of cold methanol. After drying 810 mg (63.5%) of the pure substance were obtained; m.p. 180°C (dec.), (alpha)$^{20}$ −87° (c 1.00, $CHCl_3$). IR (Nujol) 3580 (OH), 2170 (N=C), 1662, 1640, 1620, 1605 (C=C + Ar), 1345, 1165 ($SO_2$)cm$^{-1}$. $^1$H NMR ($CDCl_3$) delta 0.8—3.3 (m), 1.17 (s), 1.24 (s), 2.42 (s), 3.50 (s, 3H), 4.05—4.60 (m, 1H), 5.10 (s, br, 2H), 7.21, 7.32, 7.67, 7.78 (ABq, 4H).

### Example 7

Preparation of 3-methoxy-17-(isocyano-p-methylphenylsulfonylmethylene)androsta-3,5-dien-11-one.

3-Methoxyandrosta-3,5-diene-11,17-dione (785 mg, 2.5 mmol) was treated with the same chemicals as described in Example 1a, however using half the quantities mentioned there. After crystallization from 10 ml of methanol 875 mg (71%) of the isocyano compound are obtained; m.p. 195—205°C (dec.). Further purification by two crystallizations from $CH_2Cl_2$/methanol yielded a substance having a m.p. of about 220° (dec.) and an (alpha)$^{20}$ of −86.5° (c 1.00, $CHCl_3$). IR (Nujol) 2150 (N=C), 1705 (C=O), 1655, 1635, 1615 (C=C), 1595 (Ar), 1340, 1170 ($SO_2$) cm$^{-1}$. $^1$H NMR ($CDCl_3$) delta 0.7—3.8 (m), 0.92 (s), 1.12 (s), 2.45 (s), 3.50 (s), 4.85—5.30 (m, 2H), 7.19, 7.33, 7.62, 7.77 (ABq, 4H). Anal. calcd. for $C_{29}H_{33}NO_4S$ (491.65): C 70.85, H 6.77, N 2.85, S 6.52; found C 70.9, H 6.8, N 2.7, S 6.6%.

### Example 8a

Preparation of 1-alpha,2-alpha-methylene-6-chloro-17-(formamido-p-methylphenylsulfonylmethylene)androsta-4,6-dien-3-one.

Potassium-t-butoxide (412 mg, 3.68 mmol) was added to dry THF (30 ml). The suspension was cooled to −40°C under nitrogen. Then TosMIC (575 mg, 2.94 mmol) was added and after its solution the temperature was lowered to −75°C, followed by the addition of 6-alpha-chloro-1-alpha,2-alpha-methylene-androsta-4,6-diene-3,17-dione (810 mg, 2.45 mmol). After 5 hours stirring TosMIC was no longer present and the formamide compound was isolated. The resulting substance had a m.p. of 259—260°C. $^1$H ($CDCl_3$) delta 0.6—0.9 (m, cyclopropyl), 1.002 (s, 3H), 1.204 (s, 3H), 2.46 (s, 3H) 6.3 (m, 2H) 7.3—8.4 (m, 6H).

### Example 8b

Preparation of 1-alpha,2-alpha-methylene-6-chloro-17-(isocyano-p-methylphenylsulfonylmethylene)androsta-4,6-dien-3-one.

The formamide prepared according to example 8a (300 mg) was dissolved in 6 ml of THF and cooled to −20°C under nitrogen. Then triethylamine (0.8 ml) and $POCl_3$ (0.11 ml) were added, followed by stirring during half an hour at −20°C. The isocyanide was isolated and purified according to example 1a. The resulting pure substance had an m.p. of 144—151°C (browning at 118°C). IR ($CHCl_3$) 2110 (N=C), 1660 (C=O), 1615, 1601, (C=C), 1345, 1160 ($SO_2$) cm$^{-1}$. $^1$H NMR ($CDCl_3$) delta 0.6—0.9 (m, cyclopropyl), 1.025 (s, 3H), 1.200 (s, 3H), 2.43 (s, 3H), 6.16 (m, 2H), 7.31—7.77 (m, 4H).

### Example 9

Preparation of 3,3-ethylenedithio-17-(isocyano-p-methylphenylsulfonylmethylene)androsta-4-ene.

Potassium-t-butoxide (464 mg, 4.14 mmol) was added to dry THF (25 ml) and cooled to −60°C under nitrogen. Then TosMIC (0.659 g, 3.34 mmol) was added. After 10 minutes 3,3-ethylenedithioandrost-4-en-17-one (1 g, 2.76 mmol) dissolved in 5 ml THF was added followed by another 5 ml of THF. After two hours stirring at −60/−30°C acetic acid (0.24 ml, 4.2 mmol) was added at −40°C. After 10 minutes triethylamine (4.14 ml) and $POCl_3$ (0.55 ml) were added and the mixture was stirred during one hour (during the addition of the dehydrating agents the temperature raised to −10°C, stirring in a bath of 0°C). In order to complete the dehydration the same quantities of triethylamine and $POCl_3$ were again added and the mixture was again stirred for an hour. Then water was added and the aqueous layer was three times extracted with $CH_2Cl_2$. The collected $CH_2Cl_2$ solutions were dried over $MgSO_4$ and filtered. After evaporation of the solvent an oil was obtained. Crystallization from 20 ml of methanol afforded 0.85 g (yield 57%) of the isocyanide

m.p. 213—216°C (dec.). IR ($CHCl_3$): 2107 (N=C), 1608, 1600 (C=C), 1337, 1155, ($SO_2$) $cm^{-1}$. $^1H$ NMR ($CDCl_3$) delta 1.03 (s, 3H), 1.10 (s, 3H), 2.45 (s, 3H), 3.30 (m, 4H), 5.50 (s, 1H) 7.2—7.95 (ABq, 4H).

### Example 10

Preparation of 3,3-ethylenedioxy-17-(isocyano-p-methylphenylsulfonylmethylene)androst-5-ene.

Potassium-t-butoxide (about 7.5 mmol) was added under nitrogen to THF (50 ml) and the mixture was cooled to −40°C. Then TosMIC (1.17 g, 6 mmol) was added and after its solution 3,3-ethylenedioxyandrost-5-ene-17-one (1.65 g, 5 mmol). The reaction mixture was stirred at −30/−40°C for two hours. Though the TosMIC was completely used, the conversion of the steroid was not complete. Complete conversion was obtained by adding two times a further portion of 200 mg of TosMIC. Then phosphorous acid (615 mg, 7.5 mmol) was added after about 20 minutes followed by addition of triethylamine (7.5 ml, 54 mmol) and $POCl_3$ (1 ml, 11 mmol). After stirring for one hour in a bath of 0°C and storing over night in a cool box the reaction mixture was poured in 300 ml of a cold 10% solution of NaCl and extracted with $CH_2Cl_2$ (one time with 100 ml, then three times with 40 ml). The collected extracts were washed with a NaCl-solution (10%) and dried over $MgSO_4$. After evaporation a semi-solid residu remained, which yielded after purification with methanol and a trace of pyridine 2.07 g (89%) of the isocyanide m.p. 183—186°C (dec.). IR ($CHCl_3$) 2105 (N=C), 1569, 1332, 1150 ($SO_2$) $cm^{-1}$. $^1H$ NMR ($CDCl_3$): delta 0.95 (s, 3H), 1.03 (s, 3H), 2.47 (s, 3H), 3.93 (s, 4H), 5.36 (m, H), 7.40, 7.88 (ABq, 4H).

### Example 11

Preparation of 3-beta-(2'-tetrahydropyranyloxy)-17-(isocyano-p-methylphenylsulfonylmethylene)androst-5-ene.

3-Beta-(2'-tetrahydropyranyloxy)-17-(formamido-p-methylphenylsulfonylmethylene)androsta-5-ene was prepared from 3-bêta-(2'-tetrahydropyranyloxy)androsta-5-en-17-one by reaction with TosMIC as described in Example 1b. $^1H$ NMR formamide ($CDCl_3$): delta. 0.887 (s, 3H), 0.977 (s, 3H), 2.41 (s, 3H), 3.3—4.1 (m, 2H), 4.68 (m, 1H), 5.30 (m, 1H), 7.2—8.2 (m, 6H). The formamide compound (300 mg, 0.53 mmol) was dissolved in 6 ml THF and cooled to −20°C under dry nitrogen. Under stirring triethylamine (0.8 ml) and $POCl_3$ (0.11 ml) were added. After 30 minutes the reaction was completed. The reaction mixture was poured into an aqueous NaOH solution (50%, cooled in ice) and extracted with $CH_2Cl_2$ (one portion of 25 ml, 3 portions of 10 ml). The collected $CH_2Cl_2$ extracts were washed with a NaCl solution (10%) and dried on $MgSO_4$. After filtrating, evaporation of the solvent and drying under vacuum the isocyanide was obtained (283 mg); m.p. 146—152°C (browning at 137°C). IR ($CHCl_3$) 2106 (N=C), 1336, 1153 ($SO_2$), 1050 (—COC—) $cm^{-1}$. $^1H$ NMR ($CDCl_3$), 0.947 (s, 3H), 1.007 (s, 3H), 2.45 (s, 3H), 3.2—4.1 (m, 2H), 4.67 (m, 1H), 5.30 (m, 1H), 7.37—7.82 (ABq, 4H).

### Example 12

Preparation of 1-alpha-methyl-3-methoxy-17-(isocyano-p-methylphenylsulfonylmethylene)-androsta-3,5-diene.

The alpha,bêta-unsaturated isocyanide was prepared according to Example 10, starting from 1-alpha-methyl-3-methoxyandrosta-3,5-dien-17-one (1.57 g, 5 mmol). After cystallization from methanol at −20°C the isocyanide was obtained (1.33 g, 54%) m.p. 157—171°C. IR ($CHCl_3$) 2108 (N=C), 1338, 1156 ($SO_2$) $cm^{-1}$. NMR ($CDCl_3$) delta 0.75 (d, 3H), 0.970 (s, 3H), 1.013 (s, 3H), 2.46 (s, 3H), 3.55 (s, 3H), 5.10 (m, 1H), 5.34 (m, 1H), 7.42—7.90 (ABq, 4H).

### Example 13

Preparation of 3-methoxy-11-alpha-hydroxy-17-(isocyano-p-methyl-phenylsulfonylmethylene)androsta-3,5-diene.

Potassium-t-butoxide (160 mg, 1.5 mmol) was suspended in THF (12 ml) and cooled to −60°C. TosMIC (234 mg, 1.2 mmol) was added, followed after 10 minutes by 3-methoxy-11-alpha-hydroxyandrosta-3,5-dien-17-one (316 mg, 1.2 mmol). The clear solution was stirred for two hours at −50°C, followed by addition of triethylamine (3 ml) and $POCl_3$ (0.4 ml). The reaction mixture was stirred for 40 minutes at −40/−50°C and poured into a mixture of water and brine. After extraction with methylene chloride at pH 7, the organic layer was dried and evaporated. Crystallization from methanol afforded the title compound (260 mg, 52%), m.p. 235°C (dec.). $^1H$ NMR ($CDCl_3$) 0.98 (s, 3H), 1.10 (s, 3H), 1.49 (s, 1H), 2.47 (s, 3H), 3.55 (s, 3H), 4.07 (m, 1H), 5.10 (s, 1H), 5.22 (m, 1H), 7.36—7.82 (m, 4H). IR ($CHCl_3$) 3596 (OH), 2100 (N=C), 1655, 1630, 1610, 1594 (C=C), 1336, 1155 ($SO_2$) $cm^{-1}$.

### Example 14

Preparation of 3-(N-morfoline)-17-(isocyano-p-methylphenylsulfonylmethylene)androsta-3,5-diene.

The title compound was prepared in the same way as described in Example 13, starting from 3-(N-morfoline)-androsta-3,5-dien-17-one (1.78 g, 5 mmol). Yield: 52%, m.p. 154—156°C. $^1H$ NMR ($CDCl_3$) 0.97 (s, 2 × 3H), 2.45 (s, 3H), 2.85—3.10 (m, 4H), 3.6—3.8 (m, 4H), 5.14 (d, 2H), 7.25, 7.41, 7.73, 7.88 (ABq, 4H). IR ($CHCl_3$) 2105 (N=C), 1600 (C=C) $cm^{-1}$, 1337, 1150 ($SO_2$) $cm^{-1}$.

Example 15

Preparation of 3-methoxy-17-(isocyano-p-methylphenylsulfonylmethylene)-19-nor-androsta-3,5-diene.

The title compound was prepared in the same way as described in Example 13, starting from 3-methoxy-19-nor-androsta-3,5-dien-17-one (725 mg, 2.5 mmol). Yield: 671 mg (55%), m.p. 163—168°C. $^1H$ NMR ($CDCl_3$ + DMSO) 1.0—3.2 (m), 0.97 (s, 3H), 2.47 (s, 3H), 3.55 (s, 3H), 5.22 (m, 2H), 7.30, 7.44, 7.74, 7.88, (ABq, 4H). IR ($CHCl_3$) 2105 (N=C), 1334, 1150 ($SO_2$) $cm^{-1}$.

Example 16

Preparation of 3-methoxy-6-chloro-17-(isocyano-p-methylphenylsulfonylmethylene)androsta-3,5-diene.

The title compound was prepared in the same way as described in Example 13, starting form 3-methoxy-6-chloro-androsta-3,5-dien-17-one (1.65 g). Yield: 1.6 g (56%), m.p. 180—181°C. $^1H$ NMR ($CDCl_3$) 0.997 (s, 6H), 2.46 (s, 3H), 3.61 (s, 3H), 5.60 (s, 1H), 7.34—7.82 (ABq, 4H). IR ($CHCl_3$) 2106 (N=C), 1645, 1618, 1598 (C=C).

Example 17

Preparation of 3-beta-methoxymethoxy-17-(isocyano-p-methylphenylsulfonylmethylene)androsta-5-ene.

The title compound was prepared in the same way as described in Example 13, starting from 3-beta-methoxymethoxy-androsta-5-en-17-one (1.68 g, 5 mmol). Yield: 0.78 g, m.p. 89—90°C. $^1H$ NMR ($CDCl_3$) 0.95 (s, 3H), 1.01 (s, 3H), 2.45 (s, 3H), 3.34 (s, 3H + 1H), 4.65 (s, 2H), 5.30 (s, 1H), 7.25, 7.40, 7.72, 7.86 (ABq, 4H). IR ($CHCl_3$) 2106 (N=C), 1335, 1147, ($SO_2$), 1597 (C=C), 1035 $cm^{-1}$.

Example 18

Preparation of 3-isobutoxy-17-(isocyano-p-methylphenylsulfonylmethylene)androsta-3,5-diene.

The title compound was prepared in the same was as described in example 1a, starting from 3-isobutoxy-androsta-3,5-dien-17-one (1.17 g; 5 mmol). Yield 1.63 g. $^1H$ NMR ($CDCl_3$) delta 0.96 (d, 6H), 0.976 (s, 6H), 2.47 (s, 3H), 3.47 (d, 2H), 5.10 (s, 1H), 5.18 (tr, 1H), 7.39, 7.81 (2 × d, 4H). IR (Nujol) 2105 (N=C), 1647, 1622 (C=C), 1331, 1148 ($SO_2$).

Example 19

Preparation of 3-methoxy-9-alpha-hydroxy-17-(isocyano-p-methylphenylsulfonylmethylene)androsta-3,5-diene.

The title compound was prepared in the same way as described in Example 1a, starting from 3-methoxy-9-alpha-hydroxyandrosta-3,5-dien-17-one (1.3 g, 4.1 mmol). Yield 1.33 g (54%), m.p. 195—197°C. $^1H$ NMR ($CDCl_3$) 0.976 (s, 3H), 1.087 (s, 3H), 2.48 (s, 3H), 2.09—3.16 (m, 2H), 3.58 (s, 3H), 5.16 (s, 1H), 5.28 (m, 1H), 7.39—7.89 (ABq, 4H). IR ($CHCl_3$) 3560, 3620 (OH), 2109 (N=C), 1651, 1669 (C=C), 1158, 1349 ($SO_2$).

Example 20a

Preparation of 3-methoxy-17-(formamido-t-butylsulfonylmethylene)androsta-3,5-diene.

t-Butylsulfonylmethylisocyanide (443 mg, 2.75 mmol) was dissolved in tetrahydrofuran and cooled to −80°C. n-Butyl lithium (1.75 ml, 1.6 N) was added. After 5 minutes t-butanol (0.28 ml, 3 mmol) was added followed by 3-methoxyandrosta-3,5-diene-17-one (0.75 g, 2.5 mmol). The temperature was raised to −40°C and stirred for four hours. Potassium-t-butoxide (0.5 g) was added, and the mixture was stirred for an additional period (0.5 h). The reaction mixture was poured into ice-water containing ammoniumchloride (20 g/l). Extraction with methylenechloride, drying and evaporation in vacuo afforded the title compound as a white solid (1.05 g, 91%). IR (Nujol) 3200 (NH), 1700 (C=O), 1655, 1635 (C=C). $^1H$ NMR ($CDCl_3$) delta 0.8—3.3 (m), 0.99 (s), 1.18 (s), 1.40 (s), 3.57 (s, 2H), 5.05—5.35 (m, 2H), 7.96, 8.18, 8.66, 8.85 (AB, 2H).

Example 20b

Preparation of 3-methoxy-17-(isocyano-t-butylsulfonylmethylene)androsta-3,5-diene.

The formamide, prepared according to example 20a, (1.05 g) was dissolved in 35 ml THF and cooled to −5°C under nitrogen. Triethylamine (3.5 ml) and $POCl_3$ (0.46 ml), were added, followed by stirring for 90 minutes at −5°C. The isocyanide was isolated and purified according to example 1a. Yield: 0.82 g (74% calculated on 17-oxo-steroid), m.p. 191—193°C (dec.). Crystallization from methanol yielded the pure compound, m.p. 195—197°C (dec.), $(alpha)^{20}$ −103° (c 1.00, $CHCl_3$). IR (Nujol) 2140 (N=C), 1655, 1635, 1610 $cm^{-1}$ (C=C). $^1H$ NMR ($CDCl_3$) delta 0.8—3.2 (m), 1.00 (s), 1.09 (s), 1.48 (s), 3.57 (s, 3H), 5.05—5.40 (m, 2H).

Example 21

Preparation of 3-methoxy-17-(isocyano-methylsulfonylmethylene)androsta-3,5-diene

The title compound was prepared according to the process described in Example 1a, starting from 3-methoxyandrosta-3,5-diene (625 mg, 2.1 mmol) and methylsulfonylmethylisocyanide (298 mg, 2.5 mmol). Yield: 700 mg (84%), m.p. 198°C (dec), $(alpha)^{20}$ −111° (c 1.00, $CHCl_3$). IR (Nujol) 2140 (N=C), 1655, 1630, 1615 (C=C), 1330, 1155, 1145 ($SO_2$). $^1H$ NMR ($CDCl_3$) delta 0.8—3.3 (m), 1.0 (s, 3H), 1.08 (s, 3H), 3.06 (s, 3H), 3.56 (s, 3H), 5.1—5.4 (m, 2H). Anal. calcd. for $C_{23}H_{31}NO_3S$ (410.568): C 68.79, H 7.78, N 3.49, S 7.98; found C 68.7, H 7.9, N 3.5, S 7.8.

Example 22

Preparation of 3-methoxy-17-(isocyano-n-decylsulfonylmethylene)androsta-3,5-diene

The title compound was prepared according to the process described in Example 1a, starting from 3-methoxyandrosta-3,5-diene (1.5 g, 5 mmol) and n-decylsulfonylmethylisocyanide (1.47 g. 6 mmol). The oil obtained was mixed with methanol, cooled to −20°C, and the white solid, thus obtained was filtered off, and dried. Yield: 2.11 g (74%), m.p. 110—113°C. IR (Nujol) 2130 (N=C), 1655, 1630, 1615 (C=C), 1335, 1170, 1155, 1140 ($SO_2$). $^1H$ NMR ($CDCl_3$) delta 0.6—3.3 (M), 0.99 (s), 1.06 (s), 1.29 (s), 3.55 (s, 3H), 5.0—5.3 (m, 2H).

Example 23

Preparation of 3-methoxy-17-(formamido-pentamethylphenylsulfonylmethylene)androsta-3,5-diene

The title compound was prepared according to the process described in Example 1a. The title compound was obtained in a low yield.

Example 24

Preparation of 3-methoxy-17-(isocyano-p-methoxyphenylsulfonylmethylene)androsta-3,5-diene

The title compound was prepared according to the process described in Example 1a, starting from 3-methoxyandrosta-3,5-diene (1.5 g, 5 mmol) and p-methoxyphenylsulfonylmethylisocyanide (1.279 g, 6 mmol). Yield 2.20 g (89%), m.p. 155—160°C (dec.). IR (Nujol) 2150 (N=C), 1660, 1635, 1600 (C=C), 1335, 1155 ($SO_2$). $^1H$ NMR ($CDCl_3$) delta 0.80—3.30 (m), 0.97 (s), 3.56 (s, 3H), 3.88 (s, 3H), 5.05—5.37 (m, 2H), 6.95, 7.10, 7.80, 7.96 (AB, 4H).

Example 25

Preparation of 3-methoxy-17-(isocyano-phenylsulfonylmethylene)androsta-3,5-diene

The title compound was prepared according to the process as described in Example 1a, starting from 3-methoxyandrosta-3,5-diene (1.5 g, 5 mmol) and phenylsulfonylmethylisocyanide (1.09 g, 6 mmol). Yield 1.55 g (67%), m.p. 150—155°C. IR (Nujol) 2145 (N=C), 1655, 1630, 1605 (C=C), 1335, 1170 ($SO_2$). $^1H$ NMR ($CDCl_3$) delta 0.77—3.22 (m), 0.95 (s), 3.49 (s, 3H), 4.93—5.28 (m, 2H), 7.28—7.98 (m, 5H).

Example 26a

Preparation of 3-methoxy-17-(formamido-p-chlorophenylsulfonylmethylene)androsta-3,5-diene

The title compound was prepared according to the process as described in Example 20a, starting from 3-methoxyandrosta-3,5-diene (1.35 g, 4.5 mmol) and p-chlorophenylsulfonylmethylisocyanide (1.08 g, 5 mmol). Yield: 2.30 g (99%). IR (Nujol) 1700 (C=O), 1660, 1635, 1590 (C=C), 1325, 1150 ($SO_2$). $^1H$ NMR ($CDCl_3$) delta 0.55—2.98 (m), 0.80 (s), 3.21 (s, 3H), 4.50—4.87 (m, 2H), 6.50—7.50 (m, 6H).

Example 26b

Preparation of 3-methoxy-17-(isocyano-p-chlorophenylsulfonylmethylene)androsta-3,5-diene

The title compound was prepared according to the method described in Example 20b, starting from 2.30 g formamide. Yield 1.60 g (71%), m.p. 144—147°C (dec.). IR (Nujol) 2155 (N=C), 1660, 1635, 1615, 1585 (C=C), 1350, 1165 ($SO_2$), 770 (CCl). $^1H$ NMR ($CDCl_3$) delta 0.63—3.20 (m), 0.96 (s), 3.47 (s, 3H), 4.87—5.23 (m, 2H), 7.27, 7.40, 7.62, 7.76 (AB, 4H).

Example 27

Preparation of 3-methoxy-11-beta-hydroxy-17-(isocyano-p-methoxyphenylsulfonylmethylene)androsta-3,5-diene

The title compound was prepared according to the process as described in Example 5 starting from 2.5 mmol steroid and 3 mmol isocyanide. Yield: 1.09 g (85%). m.p. 169—172°C (dec.). IR (Nujol) 3590 (OH), 2125 (N=C), 1655, 1635, 1590 (C=C), 1325, 1155, ($SO_2$). $^1H$ NMR delta 0.81—3.30 (m), 1.22 (s), 3.56 (s, 3H), 3.88 (s, 3H), 4.33—4.63 (m, 1H), 5.08 (s, 2H), 7.08, 7.25, 7.78, 7.94 (AB, 4H). (Solvent: $CDCl_3$).

Example 28

Preparation of 17-(isocyano-p-methoxyphenylsulfonylmethylene)androsta-1,4-diene-3-one

The title compound was prepared according to the process as described in Example 3 starting from 2.5 mmol steroid and 3 mmol isocyanide. Yield 1.00 g (84%). m.p. 185—187°C. IR (Nujol) 2145 (N=C), 1660 (C=O), 1620, 1595 (C=C), 1340, 1150 ($SO_2$). $^1H$ NMR ($CDCl_3$) delta 0.78—3.27 (m), 1.02 (s), 1.25 (s), 3.89 (s, 3H). 6.09, 6.11, 6.29, 6.32 (2 × d, 2H), 6.97, 7.12, 7.80, 7.97 (AB + d, 5H).

Example 29

Preparation of 3-hydroxy-17(isocyano-p-methylphenylsulfonylmethylene)androst-5-ene

The title compound was prepared according to the method described in Example 5, starting from 2.5 mmol steroid and 3 mmol isocyanide. Yield: 130 mg (11%), m.p. 120°C (dec.). IR (Nujol) 3500 (OH), 2170 (N=C), 1610 (C=C), 1145, 1360 ($SO_2$). $^1H$ NMR ($CDCl_3$) delta 0.5—3.8 (m), 0.96 (s), 1.03 (s), 2.47 (s), 3.9—4.6 (m, 1H), 5.25—5.60 (m, 2H), 7.32, 7.46, 7.79, 7.93 (AB, 4H).

Example 30a

Preparation of 17-(formamido-p-methylphenylsulfonylmethylene)androst-4-ene-3-one

Potassium-t-butoxide (672 mg, 6 mmol) was added to dry tetrahydrofuran (40 ml) whereafter the suspension was cooled to −80°C. TosMIC (936 mg, 3.8 mmol) was added to the suspension at −80°C. After 10 minutes 2-(1'-pyrolidyl)-androsta-3,5-diene-17-one (1.36 g) was added. The mixture was stirred for 5 hours at −40°C and 2.5 hours at −35°C. Acetic acid (0.34 ml) was added followed by sodium acetate (1.2 g), acetic acid (1.2 ml) and water (6 ml). After 45 minutes the reaction mixture was poured into water and extracted with methylene chloride. After drying over $MgSO_4$, the solvent was evaporated in vacuo. Chromatography over alumine oxide (toluene, acetone 9:1) the title compound was obtained. Yield: 0.8 g, m.p. 242—245°C (dec.). IR ($CHCl_3$) 3395, 3370, (NH), 1700 (C=O), 1663 (C=O), 1320, 1140 ($SO_2$). $^1H$ NMR ($CDCl_3$) delta 0.93 (s, 3H), 1.15 (s, 3H), 2.43 (s, 3H), 5.75 (s, 1H), 7.2—8.3 (m).

Example 30b

Preparation of 17-(isocyano-p-methylsulfonylmethylene)androst-4-ene-3-one

The title compound was prepared according to the process described in Example 8b, starting from 600 mg of the formamide prepared in Example 30a. Yield: 400 mg. Physical properties are as described in Example 1a.

**Claims**

1. Compounds of the general formula:

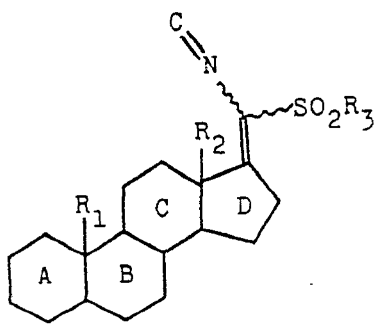

in which $R_1$ represents a hydrogen atom or an alkyl group having 1 to 4 carbon atoms, preferably a methyl group, or is absent in the case of a double bond between $C_{10}$ and $C_1$, $C_5$ or $C_9$, $R_2$ represents a hydrogen atom or an alkyl group having 1 to 4 carbon atoms, preferably a methyl group, $R_3$ represents an alkyl or dialkylamino group, or an aryl group substituted or not substituted by one or more halogen atoms, alkyl or alkoxy groups, and the ring A, B, C and D optionally contain one or more double bonds, are substituted or not substituted by one or more hydroxy groups, amino groups, oxygen atoms, halogen atoms or alkyl, alkylene, alkoxy or alkoxyalkoxy groups and are disubstituted or not disubstituted by one or more epoxy groups, methylene groups, alkylenedioxy, alkylenedithio or alkyleneoxythio groups.

2. Compounds of the general formula:

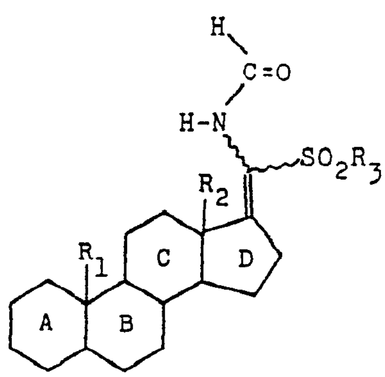

in which $R_1$ represents a hydrogen atom or an alkyl group having 1 to 4 carbon atoms, preferably a methyl group or is absent in the case of a double bond between $C_{10}$ and $C_1$, $C_5$ or $C_9$, $R_2$ represents a hydrogen atom or an alkyl group having 1 to 4 carbon atoms, preferably a methyl group, $R_3$ represents an alkyl or dialkylamino group, or an aryl group substituted or not substituted by one or more halogen atoms, alkyl or alkoxy groups, and the ring A, B, C and D optionally contain one or more double bonds, are substituted or not substituted by one or more hydroxy groups, amino groups, oxygen atoms, halogen atoms or alkyl, alkylene, alkoxy or alkoxyalkoxy groups and are disubstituted or not disubstituted by one or more epoxy groups, methylene groups, alkylenedioxy, alkylenedithio or alkyleneoxythio groups.

3. Compounds as defined in claim 1 or 2, where $R_3$ represents an alkyl group having 1 to 10 carbon atoms or $R_3$ represents a phenyl or naphthyl group optionally substituted by a halogen atom, one or more alkyl groups or an alkoxy group, preferably $R_3$ represents a phenyl, p-methoxyphenyl or p-methylphenyl group.

4. Compounds according to claims 1—3 in which the rings A, B, C and D contain one or more double bonds characterized in that these double bonds are present between $C_1$ and $C_2$, $C_3$ and $C_4$, $C_4$ and $C_5$, $C_5$ and $C_6$, $C_6$ and $C_7$, $C_9$ and $C_{11}$ and/or $C_{11}$ and $C_{12}$.

5. Compounds according to claims 1—4, in which the rings A, B, C and D are substituted by one or more 3-, 9-, 11-, 12- or 14-hydroxy groups, and/or by an oxygen atom at $C_3$, $C_{11}$ or $C_{12}$ and/or by one or more 6-, 9-, or 11-fluorine, chlorine or bromine atoms, and/or by a 1- or 6-methyl group, and/or by a 3-, 9- or 11-alkoxy group containing 1—4 carbon atoms, and/or by a 3- or 11-alkoxyalkoxy group.

6. Compounds according to claims 1—5, in which the rings A, B, C and D are disubstituted by an epoxy group at $C_1$ and $C_2$ or $C_9$ and $C_{11}$ and/or by a methylene group attached to $C_1$ and $C_2$ and/or by a 3,3-alkylenedioxy, a 3,3-alkylenedithio or a 3,3-alkyleneoxythio group.

7. Process for the preparation of the 17-(isocyanosulfonylmethylene)-steroids by reacting a ketone with a sulfonylmethylisocyanide, followed by dehydration of the resulting formamide, characterized in that the ketone is a 17-oxo-steroid.

8. Process for the preparation of 17-(formamidosulfonylmethylene)-steroids by reacting a ketone with a sulfonylmethylisocyanide, characterized in that the ketone is a 17-oxo-steroid.

9. Process according to claim 7 and 8 in which the 17-oxo-steroid has the substituents as defined in claims 1—6.

10. Process for the preparation of 17-(isocyanosulfonylmethylene)-steroids, characterized in that 17-(formamido-sulfonylmethylene)-steroids are dehydrated.

**Patentansprüche**

1. Verbindungen der allgemeinen Formel:

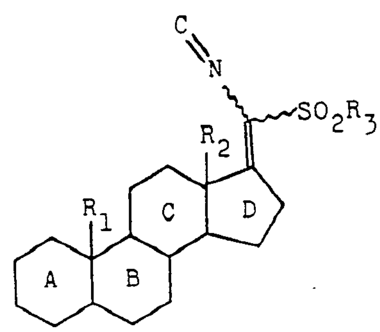

worin $R_1$ ein Wasserstoffatom oder eine Alkylgruppe mit 1 bis 4 Kohlenstoffatomen, vorzugsweise eine Methylgruppe, bedeutet oder im Falle einer Doppelbindung zwischen $C_{10}$ und $C_1$, $C_5$ oder $C_9$ abwesend ist, $R_2$ ein Wasserstoffatom oder eine Alkylgruppe mit 1 bis 4 Kohlenstoffatomen, vorzugsweise eine Methylgruppe, bedeutet, $R_3$ eine Alkyl- oder Dialkylaminogruppe oder eine Arylgruppe, die substituiert oder nicht substituiert ist durch ein oder mehrere Halogenatome, Alkyl- oder Alkoxygruppen, bedeutet und die Ringe A, B, C und D gegebenenfalls eine oder mehrere Doppelbindungen enthalten, substituiert oder nicht substituiert sind durch eine oder mehrere Hydroxylgruppen, Aminogruppen, Sauerstoffatome, Halogenatome oder Alkyl-, Alkylen-, Alkoxy- oder Alkoxyalkoxygruppen und disubstituiert oder nicht disubstituiert sind durch eine oder mehrere Epoxygruppen, Methylengruppen, Alkylendioxy-, Alkylendithio- oder Alkyloxythiogruppen.

2. Verbindungen der allgemeinen Formel:

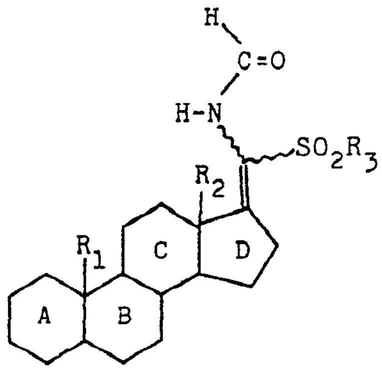

worin $R_1$ ein Wasserstoffatom oder eine Alkylgruppe mit 1 bis 4 Kohlenstoffatomen, vorzugsweise eine Methylgruppe, bedeutet oder im Falle einer Doppelbindung zwischen $C_{10}$ und $C_1$, $C_5$ oder $C_9$ abwesend ist, $R_2$ ein Wasserstoffatom oder eine Alkylgruppe mit 1 bis 4 Kohlenstoffatomen, vorzugsweise eine Methylgruppe, bedeutet, $R_3$ eine Alkyl- oder Dialkylaminogruppe oder eine Arylgruppe, die substituiert oder nicht substituiert ist durch ein oder mehrere Halogenatome, Alkyl- oder Alkoxygruppen, bedeutet und die Ringe A, B, C und D gegebenenfalls eine oder mehrere Doppelbindungen enthalten, substituiert oder nicht substituiert sind durch eine oder mehrere Hydroxylgruppen Aminogruppen, Sauerstoffatome, Halogenatome oder Alkyl-, Alkylen-, Alkoxy- oder Alkoxyalkoxygruppen und disubstituiert oder nicht disubstituiert sind durch eine oder mehrere Epoxygruppen, Methylengruppen, Alkylendioxy-, Alkylendithio- oder Alkylenoxythiogruppen.

3. Verbindungen nach Anspruch 1 oder 2, worin $R_3$ eine Alkylgruppe mit 1 bis 10 Kohlenstoffatomen bedeutet oder $R_3$ eine Phenyl- oder Naphthylgruppe, die gegebenenfalls durch ein Halogenatom, eine oder mehrere Alkylgruppen oder eine Alkoxygruppe substituiert ist, bedeutet und $R_3$ vorzugsweise eine Phenyl-, p-Methoxyphenyl- oder p-Methylphenylgruppe bedeutet.

4. Verbindungen nach den Ansprüchen 1 bis 3, worin die Ringe A, B, C und D eine oder mehrere Doppelbindungen enthalten, dadurch gekennzeichnet, dass diese Doppelbindungen zwischen $C_1$ und $C_2$, $C_3$ und $C_4$, $C_4$ und $C_5$, $C_5$ und $C_6$, $C_6$ und $C_7$, $C_9$ und $C_{11}$ und/oder $C_{11}$ und $C_{12}$ vorhanden sind.

5. Verbindungen nach den Ansprüchen 1 bis 4, worin die Ringe A, B, C und D durch eine oder mehrere 3-, 9-, 11-, 12- oder 14-Hydroxylgruppen und/oder durch ein Sauerstoffatom an $C_3$, $C_{11}$ oder $C_{12}$ und/oder durch ein oder mehrere 6-, 9- oder 11-Fluor-, -Chlor- oder -Bromatome und/oder durch eine 1- oder 6-Methylgruppe und/oder durch eine 3-, 9- oder 11-Alkoxygruppe, die 1 bis 4 Kohlenstoffatome enthält, und/oder durch eine 3- oder 11-Alkoxyalkoxygruppe substituiert sind.

6. Verbindungen nach den Ansprüchen 1 bis 5, worin die Ringe A, B, C und D durch eine Epoxygruppe an $C_1$ und $C_2$ oder $C_9$ und $C_{11}$ und/oder durch eine Methylengruppe, die an $C_1$ und $C_2$ gebunden ist, und/oder durch eine 3,3-Alkylendioxy-, eine 3,3-Alkylendithio- oder eine 3,3-Alkylenoxythiogruppe disubstituiert sind.

7. Verfahren zur Herstellung der 17-(Isocyanosulfonylmethylen)-steroide durch Umsetzung eines Ketons mit einem Sulfonylmethylisocyanid, gefolgt von Wasserabspaltung aus dem resultierenden Formamid, dadurch gekennzeichnet, dass das Keton ein 17-Oxo-steroid ist.

8. Verfahren zur Herstellung von 17-(Formamidosulfonylmethylen)-steroiden durch Umsetzung eines Ketons mit einem Sulfonylmethylisocyanid, dadurch gekennzeichnet, dass das Keton ein 17-Oxo-steroid ist.

9. Verfahren nach Anspruch 7 und 8, worin das 17-Oxo-steroid die in den Ansprüchen 1 bis 6 definierten Substituenten hat.

10. Verfahren zur Herstellung von 17-(Isocyanosulfonylmethylen)-steroiden, dadurch gekennzeichnet, dass 17-(Formamido-sulfonylmethylen)-steroide der Wasserabspaltung unterworfen werden.

**Revendications**

1. Composés de la formule générale:

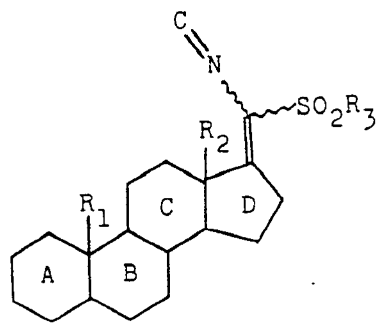

où $R_1$ représente un atome d'hydrogène ou un radical alcoyle de 1 à 4 atomes de carbone, de préférence un radical méthyle, ou est absent dans le cas d'une double liaison entre $C_{10}$ et $C_1$, $C_5$ ou $C_9$, $R_2$ représente un atome d'hydrogène ou un radical alcoyle de 1 à 4 atomes de carbone, de préférence un radical méthyle, $R_3$ représente un radical alcoyle ou dialcoylamino ou un radical aryle substitué ou non substitué par un ou plusieurs atomes d'halogène ou radicaux alcoyle ou alcoxy et les cycles A, B, C et D contiennent éventuellement une ou plusieurs doubles liaisons, sont substitués ou non substitués par un ou plusieurs radicaux hydroxyle, radicaux amino, atomes d'oxygène, atomes d'halogène ou radicaux alcoyle, alcoylène, alcoxy ou alcoxyalcoxy et sont disubstitués ou non disubstitués par un ou plusieurs radicaux époxy, radicaux méthylène, radicaux alcoylènedioxy, alcoylènedithio ou alcoylèneoxythio.

2. Composés de la formule générale:

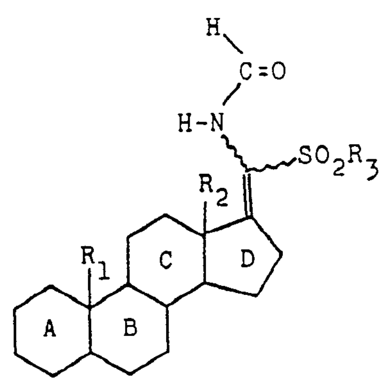

où $R_1$ représente un atome d'hydrogène ou un radical alcoyle de 1 à 4 atomes de carbone, de préférence un radical méthyle, ou est absent dans le cas d'une double liaison entre $C_{10}$ et $C_1$, $C_5$ ou $C_9$, $R_2$ représente un atome d'hydrogène ou un radical alcoyle de 1 à 4 atomes de carbone, de préférence un radical méthyle, $R_3$ représente un radical alcoyle ou dialcoylamino ou un radical aryle substitué ou non substitué par un ou plusieurs atomes d'halogène ou radicaux alcoyle ou alcoxy et les cycles A, B, C et D contiennent éventuellement une ou plusieurs doubles liaisons, sont substitués ou non substitués par un ou plusieurs radicaux hydroxyle, radicaux amino, atomes d'oxygène, atomes d'halogène ou radicaux alcoyle, alcoylène, alcoxy ou alcoxyalcoxy et sont disubstitués ou non disubstitués par un ou plusieurs radicaux époxy, radicaux méthylène, radicaux alcoylènedioxy, alcoylènedithio ou alcoylèneoxythio.

3. Composés suivant la revendication 1 ou 2, dans lesquels $R_3$ représente un radical alcoyle de 1 à 10 atomes de carbone ou $R_3$ représente un radical phényle ou naphthyle éventuellement substitué par un atome d'halogène, un ou plusieurs radicaux alcoyle ou un radical alcoxy, et de préférence $R_3$ représente un radical phényle, p-méthoxyphényle ou p-méthylphényle.

4. Composés suivant les revendications 1—3, dans lesquels les cycles A, B, C et D contiennent une ou plusieurs doubles liaisons, caractérisés en ce que ces doubles liaisons sont présentes entre $C_1$ et $C_2$, $C_3$ et $C_4$, $C_4$ et $C_5$, $C_5$ et $C_6$, $C_6$ et $C_7$, $C_9$ et $C_{11}$ et/ou $C_{11}$ et $C_{12}$.

5. Composés suivant les revendications 1—4, dans lesquels les cycles A, B, C et D sont substitués par un ou plusieurs radicaux 3-, 9-, 11-, 12- ou 14-hydroxyle et/ou par un atome d'oxygène en $C_3$, $C_{11}$ ou $C_{12}$ et/ou par un ou plusieurs atomes 6-, 9-, ou 11-fluoro-, -chloro ou -bromo, et/ou par un radical 1- ou 6-méthyle et/ou par un radical 3-, 9- ou 11-alcoxy comptant 1 à 4 atomes de carbone, et/ou par un radical 3- ou 11-alcoxyalcoxy.

6. Composés suivant les revendications 1—5, dans lesquels les cycles A, B, C et D sont disubstitués par un radical époxy en $C_1$ et $C_2$ ou $C_9$ et $C_{11}$ et/ou par un radical méthylène uni en $C_1$ et $C_2$ et/ou par un radical 3,3-alcoylènedioxy, 3,3-alcoylènedithio ou 3,3-alcoylèneoxythio.

7. Procédé de préparation des 17-(isocyanosulfonylméthylène)-stéroïdes par réaction d'une cétone avec l'isocyanure de sulfonylméthyle, puis par déshydratation du formamide résultant, caractérisé en ce que la cétone est un 17-oxo-stéroïde.

8. Procédé de préparation de 17-(formamidosulfonylméthylène)-stéroïdes par réaction d'une cétone avec l'isocyanure de sulfonylméthyle, caractérisé en ce que la cétone est un 17-oxo-stéroïde.

9. Procédé suivant la revendication 7 ou 8, dans lequel le 17-oxo-stéroïde porte les substituants tels que définis dans les revendications 1—6.

10. Procédé de préparation de 17-(isocyanosulfonylméthylène)-stéroïdes, caractérisé en ce que des 17-(formamido-sulfonylméthylène)-stéroïdes sont déshydratés.